# EUROPEAN PATENT APPLICATION

(11) **EP 1 388 295 A1**
(43) Date of publication of application: **11.02.2004**
(21) Application number: 02017803.4
(22) Date of filing: 07.08.2002
(51) Int. Cl.: A23L 1/164, A21D 2/16, A23L 1/30

(54) **Cereal based food product comprising DHA**

(71) Applicant: NESTEC S.A., 1800 Vevey (CH)
(72) Inventor: Sievert, Birgit, 1066 Epalinges (CH)
(74) Representative: Vergani, Diego

(57) **Abstract**

The invention relates to a cereal based food product, preferably shaped as a bar, having a water activity between 0.2 and 0.4 and comprising encapsulated DHA and one or more citrus flavors. The food product according to the present invention has no off-taste and odor.

## Description

### Field of the invention

The present invention relates to cereal based food products comprising DHA (decosahexanoic acid), without fish off-tastes and odors.

### Background of the invention

Docosahexaenoic acid (DHA), a long-chain omega-3 polyunsaturated fatty acid, is important throughout pregnancy and lactation for the health of both the mother and her fetus/infant. A recent National Institutes of Health workshop of fatty acid experts recognizing the importance of maternal DHA intake recommended 300mg/day of DHA as adequate intake for pregnant and lactating women. Some of the research prompting this recommendation includes:
- Maternal DHA levels decline significantly in the last trimester, the period during which much maternal DHA is transferred from mother to fetus,
- In preterm infants, DHA levels in the umbilical artery wall, which reflect the long-term fetal DHA status, have been positively correlated to newborn head circumference, weight and length,
- In a dietary study of 119 pregnant or lactating women in the United States, the average intake of DHA was 54mg/day, only 18% of that recommended by experts. Less than 2%of these women met the recommended DHA average intake,
- Increasing maternal DHA intake during pregnancy, through diet or supplements, increases maternal and newborn DHA levels,
- DHA was cited as the likely component of breast milk affecting significant increases in cognitive outcomes of breast-fed infants through the first eighteen years of life,
- Even up to two years of age, breast-fed infants have higher skeletal muscle DHA and lower blood glucose levels than formula-fed infants,
- At 6 weeks postpartum, maternal DHA levels remain lower than levels of non-pregnant women,
- Reported DHA levels in breast milk of American women 15-26 are lower than what is recommended for formula-fed infants by a joint expert committee of the World Health Organization and the Food and Agriculture Organization,
- During lactation, increasing maternal intake of DHA with dietary supplements improves maternal, breast milk and infant DHA levels.

To answer this well known and health-concerning problem, some companies have launched products, such as cereal bars, containing DHA. For example, Arkopharma Laboratories have launched snack bars (Gouter Vitalité®) for pregnant women, enriched with vitamins, minerals, essential nutrients, calcium, zinc, iron, magnesium, phosphorous, 10 vitamins and folic acid. Each bar contains 65 calories and it is recommended that one to two should be eaten each day. The ingredients include, among others, rice flour, corn flour, vegetable fatty matter, calcium phosphate, saccharose, salt, magnesium oxide, flavoring, vitamin C, DHA, vitamin E, vitamin PP, antioxidant (palmitate ascorbate) and natural tocopherols.

WO 0072842 to KV Pharmaceutical Company discloses a composition comprising a first fatty acid such as linoleic or linolenic acid in a range of 10 to 1000 mg, a second fatty acid such as DHA in a range of 10 to 1000 mg, vitamin C or a derivative thereof (i.e. ascorbate) in a range of 25 to 500 mg, and further vitamins and minerals. The composition disclosed may be in the form of any acceptable dosage forms, such as health bars, and may be in the form of cereals.

However, DHA is well known to have a very strong odor and off-taste of fish oil, rejected by the consumer. Numerous attempts have been made to mask the off taste of fish oil or DHA. For example, EP 296117 to Warner- Lambert Co proposes to render unpleasant tasting edible oil palatable by adding a sensory masking agent. The sensory masking agent can be a taste-masking agent such as anethole, dihydroanethole, eugenol, vanillin, ethylvanillin, ethyl maltol. It can also be an artificial or natural odor masking agent, such as lime, lemon, orange, pineapple, grapefruit, cinnamon, clove, bay, allspice, anise, wintergreen, spearmint, benzaldehyde or cherry.

Furthermore, the use of ascorbic acid or its derivatives is a well-known method for preventing oxidation of fish oil or DHA. JP 07107938 to Saneigen FFI KK discloses an emulsion composition for food, pharmaceuticals, cosmetics and pet food containing docosahexanoic acid and vitamin C, for long-term storage, avoiding odor change or rapid oil oxidation of e.g. purified palm oil.

However, none of the prior attempts to mask or to remove the strong off-tastes and odors of DHA have been successful, and edible products containing DHA are still rejected by the consumer because of this long lasting, strong and very unpleasant fish off-taste.

### Summary of the invention

We have now surprisingly found that it is possible to obtain cereal based food products comprising DHA, even at high concentrations, without off-taste. The cereal based food product according to the invention has a water activity between 0.2 and 0.4 and comprises encapsulated DHA and one or more citrus flavors.

### Detailed description of the invention

We have found that the off-tastes and odors of the DHA contained in the cereal based food product according to the invention completely disappear, and this applies for relatively small contents of DHA, such as 100 mg DHA per 100 grams of cereal based food product, for example, as well as for very high contents of DHA, such as 1300 mg and even up to 2200 mg or more DHA per 100 grams of cereal based food product. This allows to strongly decrease the amount of product the intended consumer has to ingest daily to meet the recommended intake of about 600 mg DHA/day. As the intended consumer is usually a pregnant or lactating woman who, in most cases, have to look closely after her diet, and especially after her daily calorie intake, eating a small number of cereal based food product, and still ingesting a high amount of DHA, is a good answer to her needs.

According to the present invention, the flavors are preferably extracted from the Rutaceae family (Sapindales order), and most preferably from the Citrus genus. This includes, according to the invention, flavors from species *Citrus aurantifolia (Christm.)* Swingle (lime), *Citrus aurantium L.* (sour orange), *Citrus limetta Risso* (bitter orange), *Citrus limon* (L.) Burm. F. (lemon), *Citrus limonia* Obsbeck (pro sp.) (mandarin lime), *Citrus maxima* (Burm. F.) Merr (shaddock), *Citrus medica* L. (citron), *Citrus paradisi* Macfad. (pro sp.) (grapefruit), *Citrus reticulata* Blanco (tangerine), and *Citrus sinensis* (L.) Osbeck (sweet orange). This aspect of the invention can be understood as comprising flavors of one or more of the aforementioned species.
According to the present invention, the flavors are preferably chosen among the group consisting of orange flavor, lemon flavor, grapefruit flavor and mixtures thereof.
Examples of particularly suitable flavors are lemon tertrarome liquid 987317 (catalogue number) and orange tetrarome liquid 987431 (catalogue number), both by Firmenich SA, Geneva, Switzerland.

The citrus flavor content of the cereal based product according to the present invention may be comprised between 0.01 to 0.20 wt-%, preferably 0.07 to 0.17 wt-%, most preferably 0.10 to 0.15 wt-%, and in a most preferred embodiment 0.13 wt-%. The term "wt-%" has to be understood, throughout the present patent application, as the amount (in grams) of a certain ingredient per 100 g of the final product.

The citrus flavors are preferably added into the cereal based food product either in powdered form or associated with alcohol. They can also be added associated with water or water-containing liquids as far as the water activity value of the final cereal based product is maintained between 0.2 and 0.4.

It has to be understood that in addition to the citrus flavors, other flavors can be added such as, for example, honey flavor, fruit flavor, chocolate flavor, caramel flavor, nut flavor, almond flavor, yogurt flavor and mixtures thereof.

Water activity can be defined as the ratio of the water vapor pressure of a product to the vapor pressure of pure water at the same temperature. If the target water activity is exceeded, free water will migrate into the cereals. Moisture transfer may generate loss of the crispy texture, a mat appearance, the development of stale notes/off-tastes and may accelerate fat oxidation reactions and, therefore, the cereal based product will not be shelf stable. The cereal based food product according to the present invention shows a water activity comprised between 0.2 and 0.4, preferably 0.25 to 0.38, most preferably 0.30 and 0.35 and, in a most preferred embodiment,0.33.

DHA must be encapsulated into an edible component forming a closed barrier between DHA and the rest of the product or the atmosphere. Accordingly, encapsulating DHA in a matrix is not sufficient, for the purpose of the present invention. Examples of encapsulation fulfilling the requirements of the present invention are encapsulations in sugar, proteins, fats glycerol, or a mixture of monosaccharides and alcohol such as propanetriol, for example. According to a preferred embodiment of the present invention, the effective amount of DHA can vary up to 2200 milligrams of DHA per 100 grams of cereal based food product. The effective amount of DHA is defined on a 100% basis and, therefore, it merely includes the amount of pure DHA, and not that of pure DHA plus the encapsulating material.

It is also possible to add some other components to the cereal based food product according to the invention in order to further improve the organoleptic and/or nutritional characteristics of the product. These components may be chosen among glycerol, honey, prebiotics, probiotics, antioxidants, oxygen absorbers and others.

"Prebiotic" means a substance or compound which is fermented by the intestinal flora of a pet and/or a human and hence promotes the growth or development of bifido- and lactic-bacteria in the gastro-intestinal tract at the expense of pathogenic bacteria.
Suitable prebiotics include oligosaccharides, such as inulin and its hydrolysis products commonly known as fructooligosaccharides, galacto-oligosaccarides, xylooligosaccharides or oligo derivatives of starch. The prebiotics may be provided in any suitable form. For example, the prebiotic may be provided in the form of plant material which contains the prebiotic. Suitable plant materials includes asparagus, artichokes, onions, wheat or chicory, or residues of these plant materials. Alternatively, the prebiotic may be provided as an inulin extract. Extracts from chicory are particularly suitable. Suitable inulin extracts are commercially available.

"Probiotic micro-organism" means a micro-organism which beneficially affects a host by improving its intestinal microbial balance (Fuller, R; 1989; J. Applied Bacteriology, 66: 365-378).
The probiotic micro-organism may be selected from one or more micro-organisms suitable for animal and/or human consumption and which is able to improve the microbial balance in the intestine. Examples of suitable probiotic micro-organisms include yeasts such as *Saccharomyces, Debaromyces, Candida, Pichia* and *Torulopsis,* moulds such as *Aspergillus, Rhizopus, Mucor,* and *Penicillium* and *Torulopsis* and bacteria such as the genera *Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus* and *Lactobacillus.* Specific examples of suitable probiotic micro-organisms are: *Saccharomyces cereviseae. Bacillus coagulans, Bacillus licheniformis, Bacillus subtilis, Bifidobacterium bifidum, Bifidobacterium infantis, Bifidobacterium longum, Enterococcus faecium, Enterococcus faecalis, Lactobacillus acidophilus, Lactobacillus alimentarius, Lactobacillus casei* subsp. *casei, Lactobacillus casei Shirota, Lactobacillus curvatus, Lactobacillus delbruckii* subsp. *lactis, Lactobacillus farciminus, Lactobacillus gasseri, Lactobacillus helveticus, Lactobacillus johnsonii, Lactobacillus reuteri, Lactobacillus rhamnosus (Lactobacillus* GG), *Lactobacillus sake, Lactococcus lactis, Micrococcus varians, Pediococcus acidilactici, Pediococcus pentosaceus, Pediococcus acidilactici, Pediococcus halophilus, Streptococcus faecalis. Streptococcus thermophilus, Staphylococcus carnosus,* and *Staphylococcus* *xylosus.* The probiotic micro-organisms may be in powdered, dried form; especially in spore form for micro-organisms which form spores. Further, if desired, the probiotic micro-organism may be encapsulated to further increase the probability of survival; for example in a sugar matrix, fat matrix or polysaccharide matrix.

Antioxidants inhibit oxidation by molecular oxygen, and are commonly used to delay fat staling. Primary antioxidants act by blocking free radicals such as peroxide radicals, which are responsible for the first oxidation step of unsaturated fatty acids. Secondary antioxidants act either by chelating metallic ions, which are the oxidation catalysts, or by inhibiting lipoxygenases. Suitable antioxidants are, for example, L ascorbic acid, 1 sodium ascorbate, 1 calcium ascorbate, ascorbyle palmitate, alpha, delta or gamma tocopherols, BHA (butylhydroxyanisol), BHT (butylhydroxytoluene), lactic acid, sodium, potassium or calcium lactates, citric acid, and more generally anti-oxygens E 300 to E 309, E 311 and E 312, E 320 to E 322, E 220 to E 224, E 226, E 270, E 325 to E 327, E 330 to 341, and E 472c (European Economic Community numerotation)

Preferably, no minerals such as iron or copper are added, as they are catalysts for fat oxidation. Oxygen absorbers, such as sodium ascorbate, can also be added to the cereal based food product comprising DHA.

The cereal based food product according to the invention can have different shapes or appearance forms. For example, it can be shaped as a stick, a cake, a macaroon, a bar or it can be in form of muesli (free cereals) or flakes. It can be consumed alone or in association with dairy products such as milk, yogurts, cottage cheeses, or the like. A preferred product appearance is a cereal bar of 23 grams.

Another aspect of the present invention relates to a packaged food product comprising a package including, under modified atmosphere, the cereal based food product described above. Preferably, the cereal based food product is shaped as a bar and the modified atmosphere contains less than 0.5 vol-% of molecular oxygen.

If the cereal based food product according to the invention has to be stored for more than a week, before being consumed, which is generally the case, it is essential to do it under modified atmosphere. The product can be packed by sealing the pouches in a glove compartment filled with inert gas. The inert gas can be any gas known by the skilled person for its use in food-containing packaging, such as N₂, CO₂ and mixtures thereof.

The longer the cereal based food product is stored, the more important is that this takes place under modified atmosphere. Indeed, if said food product is stored less than one week before its consumption, a modified atmosphere, although preferable, is not necessary.

Intermediate storage of DHA bars is also preferably done under a modified atmosphere, for example in an Atlas Lyophilisator under N₂. The primary packaging of the bars can be processed in two steps: during the first step, the bars are packed individually in an oxygen-tight pouch, and during the second step the bars are packed under protection of inert gas (for example N₂) by sealing the pouches in a glove compartment filled with N₂.

According to another aspect of the invention, there is a process for making a cereal based food product, particularly shaped as a bar, comprising DHA with no off-taste and odor, even at high concentrations of DHA. Accordingly, DHA is encapsulated by any food grade encapsulation process known by the skilled person. The encapsulation must lead up to an embedded encapsulation wherein exchanges with the other components of the bar and the atmosphere are as reduced as possible. Then, DHA is mixed with cereals such as, for example, corn flakes, rice crisps, shredded wheat, oats, millet and the like, or a combination thereof. The cereals can be of any suitable form, such as flakes, crisps, and balls, among others. Citrus flavors such as orange flavors are possibly added into the cereal mix. Separately, a binder is prepared by mixing at least one sugar with an oxygen absorber, such as sodium ascorbate and, possibly, citrus flavors. Citrus flavors are preferably added both to the cereal mix ingredients and to the binder ingredients. However, they can be added only to the cereal ingredients or only to the binder ingredients. If added both to the cereal mix and the binder, the flavors used for the cereal mix and those used for the binder are preferably the same.

The binder ingredients are pre-weighed and transferred into a cooker and mixer machine. The ingredients must be homogeneously mixed, i.e. lump-free and dissolved under stirring and target temperature and vacuum. The aim is to obtain the target water activity and then to add the DHA, sodium ascorbate and flavors at a maximum temperature of 80 °C. Preferably, the temperature throughout the whole process is comprised between 30 and 80°C, most preferably between 40 and 60°C, and in a most preferred embodiment is 50°C. This low temperature protects DHA, sodium ascorbate and flavors from oxidation and other chemical transformations.

This mix is transported to a mixer for cereal/binder blend. The binder and cereals are then homogeneously mixed and the blend is immediately used for bar forming to minimize fat oxidation, especially the oxidation of DHA.

A slab former, such as a two-roller slab former feeds the cereals and binder mass onto a belt. A compression roller assures the required height and homogeneity of the slab. A profiling roller shapes the slab into the desired shaped product stripes. After passing a cooling tunnel the product is cross cut (guillotine) and transported to the packaging area on numbered trays protected by plastic bags.

### Examples

The following examples are illustrative of some of the products and methods of making the same falling within the scope of the present invention. They are not to be considered, in any way, limiting of the invention. Changes and modifications can be made with respect to the invention. That is, the skilled person will recognize many variations in these examples to cover a wide range of formulas, ingredients, processing, and mixtures to rationally adjust the naturally occurring levels of the compounds of the invention for a variety of applications.

### Example 1: ingredient description of cereal bars containing DHA

| **Cereals** | **Functional properties** |
|---|---|
| Rice crisps | Cereal texture, taste, color |
| Corn flakes small | Cereal texture, taste, color |
| Natural Lemon flavor powder | Flavor, fish off-taste/odor masking agent |
| Natural Orange flavor powder | Flavor, fish off-taste/odor masking agent |

| **Binder ingredients** | |
|---|---|
| Glucose syrup | Binding, anti-crystallization agent, sweetness |
| Sucrose | Sweetness, filler |
| Fish oil concentrated powder | Encapsulated DHA (docosahexaenoic acid) |
| rich in DHA | source |
| Glycerol | Humectant, a_{w} decrease |
| Invert sugar syrup | Binding, anti-crystallization agent, sweetness, a_{w} decrease |
| Vegetable oil fractionated, non-hydrogenated, non-lauric | Taste, texture-lubrication |
| Honey | Taste, texture |
| Salt | Taste, a_{w} decrease |
| Sodium Ascorbate | Oxygen absorber |
| Honey flavor natural identical | Flavor |
| Natural Lemon flavor liquid | Flavor, fish off-taste/odor masking agent |
| Natural Orange flavor liquid | Flavor, fish off-taste/odor masking agent |

### Example 2: shelf life data

Shelf life tests and headspace analysis (oxidation test) were carried out at 37 °C (accelerated conditions) and at 20 °C/70 % relative humidity (rh). The products were analyzed for sensory, pentane and residual oxygen. 90 days at 37 °C correspond to a shelf life of 12 months under ambient non-tropical conditions (about 20 °C). The bars were stored in closed tins to exclude the effect of the packaging material or in the case of the modified atmosphere packaging (MAP) in the original packs. A scale from 0-10 evaluates the taste and odor. A rating < 6 indicates a non-acceptable product.

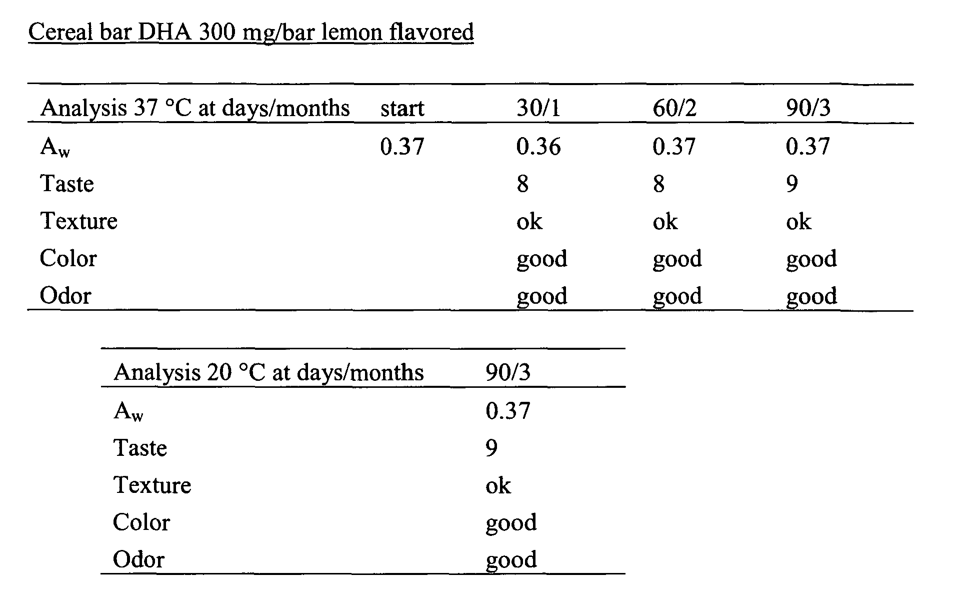

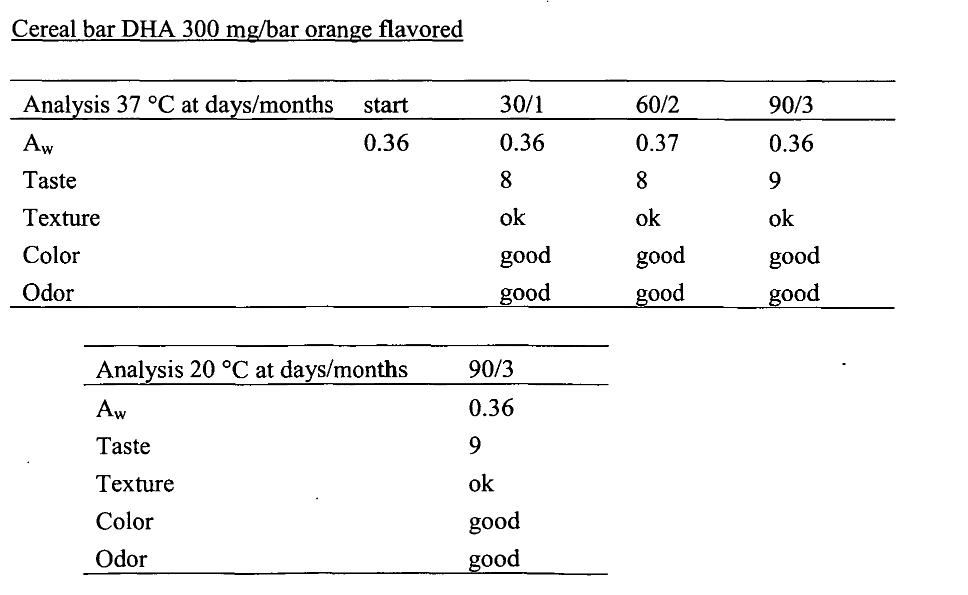

### Example 3: shelf life data of DHA bars without the combination of Citrus flavors and MAP

Series of cereal based food products with DHA 200, 300, 400 mg/bar were produced without citrus flavor and without MAP. All products showed already after 1 month shelf life at 37°C a strong fish off-taste and odor, while the water activity, moisture, pentane and residual oxygen were comparable to the reference product.

A triangular test was carried out with a DHA bar 200 mg/bar (without citrus flavored and without MAP) against a reference bar. The samples were fresh, i.e. only one week aged.

The triangular test was significant in the sense that 16 out of 21 found a difference (99% level). However, 10 out of the 16 found attributes like fruity, honey - positive attributes, and only 6 out of the 16 found a negative off-taste like metallic, rancid. Overall, only one person identified a fish off-taste in the fresh bars, i.e. only one week aged.

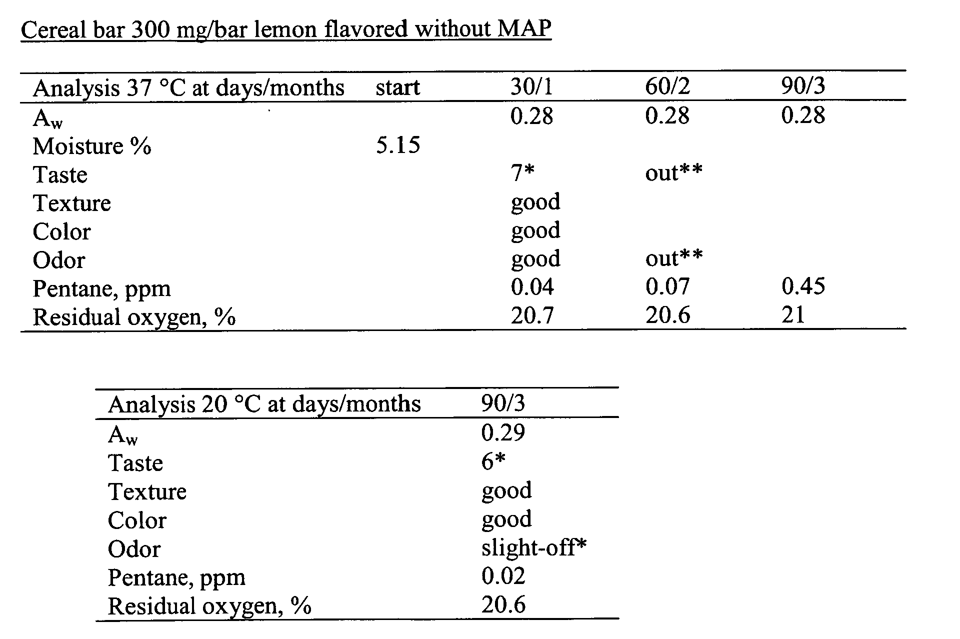

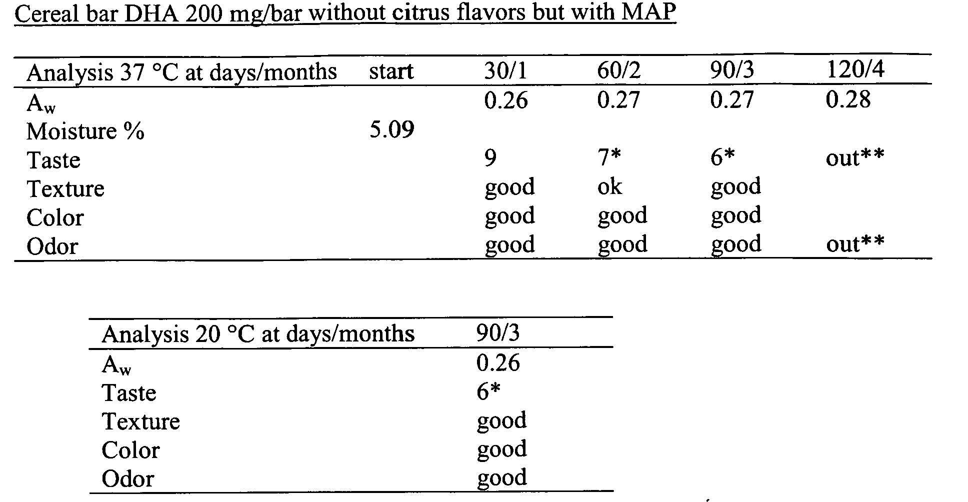

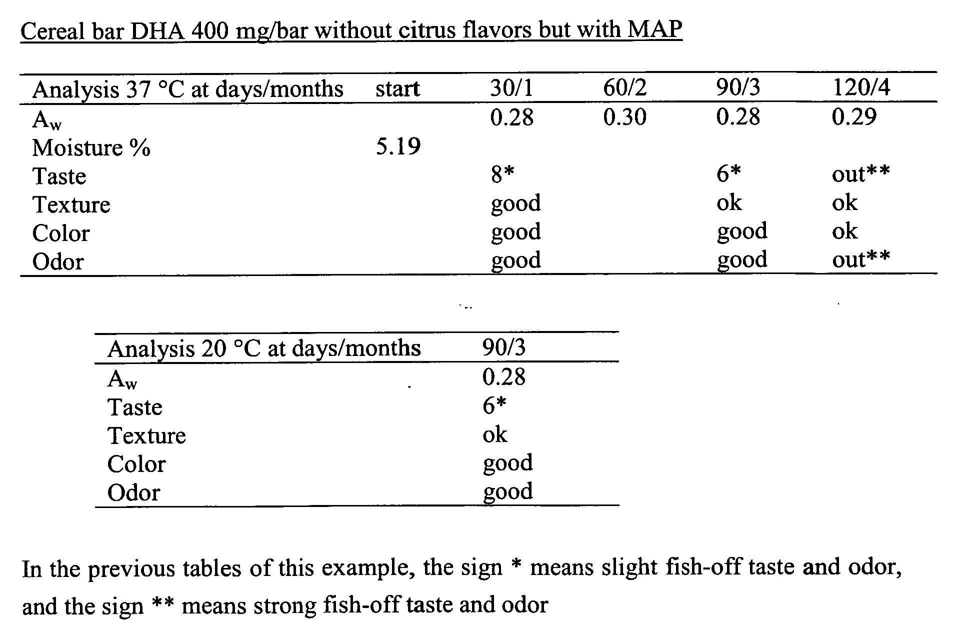

## Claims

1. Cereal based food product having a water activity between 0.2 and 0.4 and comprising encapsulated DHA and one or more citrus flavors.

2. Cereal based food product according to claim 1 wherein the effective amount of DHA does not exceed 2200 mg per 100 g food product.

3. Cereal based food product according to claim 2 wherein the effective amount of DHA does not exceed 1800 mg per 100 g food product.

4. Cereal based food product according to claim 2 wherein the effective amount of DHA is 1300 mg per 100 g food product.

5. Cereal based food product according to one of claims 1 to 4 wherein the one or more citrus flavors are present in an amount varying between 0.01 and 0.20 wt-%.

6. Cereal based food product according to claim 5 wherein the one or more citrus flavors are present in an amount varying between 0.10 and 0.15 wt-%.

7. Cereal based food product according to claim 5 wherein the on or more citrus flavors are present in an amount of 0.13 wt-%.

8. Cereal based food product according to one of claims 1 to 7 wherein the one or more citrus flavors are chosen among the group consisting of orange flavor, lemon flavor, grapefruit flavor and mixtures thereof

9. Cereal based food product according to one of claims 1 to 8 having a water activity between 0.28 and 0.38.

10. Cereal based food product according to claim 9 having a water activity of 0.33.

11. Cereal based food product according to any preceding claim further comprising at least one of glycerol, honey, prebiotics, probiotics, antioxidants and oxygen absorbers.

12. Cereal based food product according to any preceding claim wherein the food product is shaped as a stick, a cake, a macaroon, a bar or is in form of muesli or flakes.

13. Cereal based food product according to claim 12 wherein the food product is shaped as a bar.

14. Packaged food product comprising a package including a cereal based food product according to one of claims 1 to 13 under modified atmosphere.

15. Packaged food according to claim 14 wherein the modified atmosphere contains less than 0.5 vol-% of molecular oxygen.

16. Packaged food according to claim 14 or 15 wherein the cereal based food is shaped as a bar.

17. Process for making a cereal food product according to one of claims 1 to 13 comprising the steps of
(1) preparing a cereal mix by mixing cereals, encapsulated DHA and possibly one or more citrus flavors,
(2) preparing a binder possibly containing one or more citrus flavors,
(3) mixing the product obtained under (1) and the product obtained under (2) to obtain the cereal food product,
wherein at least one of the products obtained under (1) and (2) includes one or more citrus flavors and the temperature throughout the whole process does not exceed 80°C.

18. Process according to claim 17 wherein the temperature throughout the whole process does not exceed 50°C.
